# EUROPEAN PATENT SPECIFICATION

(11) **EP 1 809 250 B1**
(45) Date of publication and mention of the grant of the patent: **25.01.2017**
(21) Application number: 05812710.1
(22) Date of filing: 27.10.2005
(51) Int. Cl.: A61K 47/10, A61K 47/14, A61K 47/26, A61K 47/38, A61K 31/165, A61K 9/16, A61K 47/36, A61K 9/10

(54) **DYE-FREE PHARMACEUTICAL SUSPENSIONS COMPRISING SORBITOL AS A NON-REDUCING SWEETENER AND AT LEAST ONE ACTIVE AGENT**
FARBSTOFFFREIE PHARMAZEUTISCHE SUSPENSIONEN MIT SORBITOL ALS NICHTREDUZIERENDEM SÜSSSTOFF UND MINDESTENS EINEM WIRKSTOFF
SUSPENSIONS PHARMACEUTIQUES EXEMPTES DE COLORANT COMPRENANT DU SORBITOL EN TANT QU'EDULCORANT NON REDUCTEUR ET AU MOINS UN AGENT ACTIF

(30) Priority: 29.10.2004 US 977655
(43) Date of publication of application: 25.07.2007
(60) Divisional application: 10075198.1
(73) Proprietor: Johnson & Johnson Consumer Inc., Skillman, NJ 08558 (US)
(72) Inventor: BUEHLER, Gail K., Ambler PA19002 (US)
(74) Representative: Kirsch, Susan Edith
(86) International application number: PCT/US2005/038945
(87) International publication number: WO 2006/050101

(56) References cited:
- EP-A- 0 620 001
- EP-A- 0 717 992
- US-A- 4 361 580
- US-A- 4 427 681
- US-A- 5 409 907
- US-A- 5 658 919
- US-A- 5 688 529
- US-A1- 2003 118 654
- US-A1- 2003 191 192

## Description

### FIELD OF THE INVENTION

This invention relates to liquid pharmaceutical compositions. More particularly, the invention relates to dye-free pharmaceutical suspensions containing at least one substantially water insoluble active agent, e.g., acetaminophen.

### BACKGROUND OF THE INVENTION

Orally administered medicaments or pharmaceuticals are given to the patient in many forms, including solid forms, such as, capsules, caplets, gel caps, or tablets, and liquid forms, such as, solutions, e.g., syrups and elixirs, emulsions, or suspensions. Medicaments administered in solid form are usually intended to be swallowed whole, therefore, the often disagreeable taste of the active agent need not be taken into account in formulating the medicine, except for the provision of means to prevent the taste from being apparent during the short time the medicine is in the mouth. Such means may include the provision of an appropriately thin and quickly dissolving coating on a tablet or caplet or the use of a gelatin capsule form, (the gelatin outer shell of the capsule keeps the active agent inside until the capsule has been swallowed), or simply compressing a tablet firmly so that it will not begin to disintegrate during the short time that it is intended to be in the mouth.

Children, older persons, and many other persons, including disabled or incapacitated patients, have trouble swallowing solid forms, e.g., whole tablets and even capsules. Therefore, in cases where the dosage to be administered cannot be made into a very small tablet or capsule, it is desirable to provide the medicine either in a chewable solid form or a liquid form. For many patients, including pediatric and geriatric patients, a liquid oral dosage form is preferable over chewable dosage form because of the ready swallowability without chewing of the liquid dosage form.

A common problem associated with liquid dosage forms is the often disagreeable taste of the active agents that manifest during the time that the liquid dosage form is in the mouth prior to swallowing. While suspensions typically offer superior taste masking to other liquid forms, those skilled in the art are aware of the considerable technical difficulties in producing a stable and organoleptically acceptable suspension.

Suspensions are a two-phase system having solid substantially water insoluble active agent particles dispersed throughout liquid medium. A suspension does not encompass emulsions, which are meant to describe liquids suspended within liquid carriers or syrup formulations containing only substantially fully dissolved pharmaceutical active agents. As used herein, a "particle" may be a crystal, a granule, an agglomerate, or any undissolved solid material. The particles of the present invention preferably have a median particle size (d50%) of from about 2 to about 150 microns, more preferably from about 10 to about 100 microns. The challenges of keeping the substantially water insoluble active agent suspended, assuring stability of the substantially water insoluble active agent, and maintaining dose uniformity for a prolonged period of time, have been previously addressed. See for example, U.S. Pat. Nos. 5,409,907, and 5,374,659.

In a pharmaceutical suspension, typically at least one active agent is present substantially in the form of undissolved solid particles, i.e., the substantially water insoluble active agent. However, in any such system, a portion of such active agent may be in the dissolved state. In formulating such systems, it is advantageous to minimize the amount of drug present in the dissolved state. Minimizing the amount of active agent in solution is advantageous for both the taste and chemical and physical stability of the product.

Dyes are often added to pharmaceutical suspensions for elegance and to mask discoloration. However, it has been found that some patients develop or have allergies to or are sensitive to dyed suspension or that such suspensions stain clothing, furniture, carpeting, and the like when spilled. Therefore, dye-free suspensions are very desirable.

Coloring agents are often added to pharmaceutical liquid products to produce pharmaceutically acceptable characteristics, to provide an identifying factor and also to provide consistency among the batches of a product. Often the color of the excipients that are used to manufacture product contribute an off-color to the product. This color is often dependent on the lots of excipients and they can change on storage with no adverse effect on the product. The consumer would however, perceive the color change as having an adverse effect on the effectiveness of the product, resulting in rejection of the product.

The stability of acetaminophen (N-acetyl para-aminophenol or "APAP") is affected by formulation excipients that are needed to manufacture an acceptable product.

Thus, the challenges of manufacturing an acceptable dye-free APAP liquid product are many. In preparing an APAP suspension product, the formulator must ensure that sensory properties are acceptable and the product is acceptable to the user. In this regard, necessary flavors, sweeteners, and consistency modifiers are added to the product. In addition, the formulator must also be aware of the necessary pharmaceutical attributes that must be incorporated into the product. These include: acceptable suspension properties, stability, and microbial properties. All of these requirements complex the challenge of manufacturing an acceptable dye-free APAP suspension product.

EP 0 717 992 discloses aqueous suspension formulations for pharmaceutical applications. The suspensions disclosed have a pH from 3 to 6, preferably from 4 to 5.

US 4 361 580 disclosed aluminium ibuprofen pharmaceutical liquid suspensions.

US 2003/118654 discloses a liquid pharmaceutical composition having a pH of from 2.5 to 8. Furthermore, all examples of US 2003/118654 include invert sugar and are, therefore, not free of reducing sugars.

EP 620 001, US 5 409 907 and US 5 658 919 disclose aqueous pharmaceutical suspensions. The combination of sorbitol and sucrose is not disclosed. Furthermore the pH range of from 5.1 to 5.9 is also not disclosed. All examples have high fructose corn syrup and are therefore not free of reducing sugars.

US 4 427 681 discloses thixotropic compositions employing titanium dioxide as an opacifying agent. The compositions of US 4,427,681 do not employ a therapeutically effective amount of APAP combination in with sorbitol and sucrose and a pH range of from 5.1 to 5.9.

US 2003 0191192 discloses aqueous pharmaceutical suspensions for oral administration. There is no disclosure of suitable pH and all examples include an invert sugar and are, therefore, not free of reducing sugars.

US 5 688 529 discloses oral suspensions of mycophenolate mofetil or mycophenolic acid.

The present invention is directed to discovery of a stable dye-free aqueous APAP suspension system that achieves a palatable dosage form for both geriatric and especially pediatric applications.

### SUMMARY OF THE INVENTION

As embodied and fully described herein the present invention provides a dye-free pharmaceutical suspension composition comprising, consisting of and/or consisting essentially of:
(a) a therapeutically effective amount of APAP having an average particle size of between about 10 and about 100 microns,
(b) an effective amount of a non-reducing sweetener comprising a combination of sucrose and sorbitol;
(c) from 25 to 60 grams per 100 mL of the suspension of water;
(d) an effective amount of a suspending system;
wherein the dye-free pharmaceutical suspension has a pH of from 5.1 to 5.9 and is substantially free of a reducing sugar.

It is to be understood that both the foregoing general description and the following detailed description are exemplary, but are not restrictive, of the invention.

### DETAILED DESCRIPTION OF THE INVENTION

As used herein, the term "dye-free" is used herein as an adjective and means that a dye per se is not added to the composition of matter at any point during its manufacture or packaging.

As used herein, the term "reducing sugar" means a sugar that can chemically react with a special copper reagent known as Fehlings solution (alkaline solution), whereby the "reducing" sugar will reduce this copper solution to copper oxide (cuprous oxide). Examples of reducing sugars include, but are not limited to, corn syrup, fructose, and milk sugars.

As used herein, the term "substantially free of a reducing sugar" means less than about 4 g/100 ml of any reducing sugar.

As used herein, the term "APAP" means acetaminophen or N-acetyl para-aminophenol, including, but not limited to, pharmaceutically acceptable salts, esters, or derivatives thereof.

As used herein, the term "substantially water insoluble" refers to compositions that are insoluble, practically insoluble or only slightly soluble in water as defined by U.S. Pharmacopeia, 24th edition. These compositions require at least about 100 parts of solvent per part of said composition, for complete dissolution.

The present invention provides a dye-free suspension system particularly well suited for use in pharmaceutical suspensions. It is the applicants' discovery that a stable and pourable dye-free pharmaceutical suspension can be formed having a substantially water insoluble active agent, i.e., APAP, in suspended form, and optionally further active agents, either at least one additional substantially water insoluble active agent, at least one substantially water soluble active agent, or mixtures thereof, in either suspended, dissolved, or both forms.

The invention will now be described specifically in terms of various embodiments. One embodiment includes aqueous suspensions of the substantially water insoluble active agent APAP. APAP is a medicament used in both over-the-counter preparations and in prescription drugs for analgesic and antipyretic purposes. APAP is generally indicated for the temporary relief of minor aches and pains associated with the common cold, headache, toothaches, muscular aches, backache, for minor pain of arthritis, for the pain of menstrual cramps and for the reduction of fever. In certain embodiments, the suspension of the present invention will include APAP in suspended form, together with additional pharmaceutical active agents, which may be present in dissolved or suspended form. Reference will also be made in detail herein to other preferred embodiments of the compositions, processes and methods of the invention.

Suitable additional pharmaceutical active agents include analgesics, antiinflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

The active agent or ingredients are present in a "unit dose volume" of the aqueous suspension in a therapeutically effective amount, which is an amount that produces the desired therapeutic response upon oral administration and can be readily determined by one skilled in the art. In determining such amounts, the particular active agent being administered, the bioavailability characteristics of the active agent, the dose regime, the age and weight of the patient, and other factors must be considered, as known in the art. As used herein a "unit dose volume" of the aqueous suspension is a convenient volume for dosing the product to a patient. The dosing directions instruct the patient to take amounts that are multiples of the unit dose volume depending on, e.g., the age or weight of the patient. Typically the unit dose volume of the suspension will contain an amount of active agent(s) that is therapeutically effective for the smallest patient. For example, suitable unit dose volumes may include one teaspoonful (about 5 mL), one tablespoonful (about 15 mL), one dropperful, or one milliliter.

In one embodiment, the aqueous pharmaceutical suspension composition in accordance with the present invention includes from 0.05% to 40%,-e.g., 0.05% to 0.2%, or 1.6 to 10%, or 15 to 40% weight per volume (w/v) of at least one substantially water insoluble active agent. Amounts of active agent in this range are generally acceptable for taste modifying. It is possible that more than 40% of a substantially water insoluble active agent could be included in the suspension and be sufficiently taste masked for consumer acceptability. Suspensions containing less than 0.05% of pharmaceutical active agents are also possible.

In one embodiment, in which the first active agent is APAP, the level of active agent in the suspension is from 80 to 160 mg per 1.6 mL, or 5 to 10% w/v. In another embodiment, in which the first active agent is APAP, the level of active agent in the suspension is from about 80 to about 160 mg per teaspoonful, or 1.6 to 3.2 grams per 100 mL, or 1 to 4% w/v and having an average particle size of between 10 and 100 microns.

It has been found by the present inventor, that the unique combination of APAP having sorbitol and sucrose at a pH of from 5.1 to 5.9 produces advantageously storage stable and homogeneously dispersed suspensions of APAP.

The pharmaceutical suspension of the present invention may contain at least one additional pharmaceutical active. Such additional pharmaceutical active may be an antihistamine, an antitussive, guafenesin, and a sympathomimetic.

Antihistamine examples include those selected from the group consisting of chloropheniramine maleate, terfenadine, astemizole, diphenhydramine hydrochloride and mixtures thereof.

Antitussive examples include those selected from the group consisting of dextromethorphan HBr, diphenhydramine hydrochloride and mixtures thereof.

Sympathomimetic examples include those selected from the group consisting of pseudoephedrine hydrochloride, phenylpropanolamine and mixtures thereof.

The suspension of the present invention may also include a taste- masking composition to mask the bitter taste of the actives in the composition, particularly the suspended acetaminophen. Generally the taste-masking composition contains at least one sweetening agent and at least one flavoring agent. The flavoring agents added to the mixture should be of the type and amount desired for the particular suspension to meet the preferences dictated by the intended consumer of such suspension, e.g., pediatric or adult.

Suitable sweetening agents are non-reducing sugars, polyhydric alcohols, and high intensity sweeteners. Examples of suitable non-reducing sugars include, but are not limited to, the heterodisaccharides sucrose, lactose, raffinose, stachyose; the non-reducing homodisaccharide trehalose; and the non-reducing homo-oligosaccharides cyclomaltohexa(...deca)ose (also known as Schardinger dextrins).

The amount of sugar sweetener used in the suspension will vary depending on the degree of sweetening desired for the particular suspension. Generally the amount of sugar sweetener will be in the range of 0 to 110 grams per 100 mL of the suspension. The amount of sugar sweetener can also be in the range of from 40 grams to 100 grams per 100 mL of suspension.

Water-soluble high intensity sweeteners also may be employed in place of or in addition to sugar sweeteners. Examples of suitable high intensity sweeteners include, but are not limited to, sucralose, aspartame, saccharin, acesulfame, cyclamate, and pharmaceutically acceptable salts and combinations thereof. The amount of high intensity sweetener used in the suspension will vary depending on the degree of sweetening desired for the particular suspension. Generally the amount of high intensity sweeteners used in the suspension may vary from in the range of 0 to 5 grams per 100 mL of suspension. In embodiments employing a high intensity sweetener, such as sucralose, aspartame, acesulfame, saccharin, and pharmaceutically acceptable salts thereof, the level of high intensity sweetener is from 0 to 1 gram per 100 mL of suspension, a useful level is from 0 to 0.5 gram per 100 mL of suspension.

The pharmaceutical suspension of the present invention is substantially free of reducing sugars. Monosaccharide reducing sugars unsuitable for use in the present invention include, but are not limited to, glucose, fructose, galactose, ribose, mannose, sorbose, arabinose, and xylose. Reducing oligosaccharides unsuitable for use in the present invention include, but are not limited to, cellobiose, isomaltose, maltose, gentibiose, laminaribiose; maltotriose, maltotetrose, maltopentose, maltohexose;. Heterodisaccharides reducing sugars include, but are not limited to, lactose, lactulose, maltulose, melibiose. Reducing sugars are open-chain hydroxy aldehydes and hydroxy ketones, and are readily oxidized to form acids. The basic amino groups of proteins, peptides, and amino acids are readily added via condensation reaction to the carbonyl groups of acyclic (reducing) sugars. The Maillard reaction, a well known reaction resulting in a brown color formation, procedes via enolization of the resulting glycosylamines. A second type of sugar decomposition reaction, also resulting in formation of a brown color, results from the enolization of hydroxyaldehydes and hydroxyketones at pH less than 4 and at elevated temperatures, followed to dehydration to form furfurals (2-furaldehydes). This type of reaction is sometimes referred to as "caramelization."

Examples of suitable polyhydric alcohols for use as sweeteners in the present invention include, but are not limited to, sorbitol, mannitol, xylitol, erythritol, maltitol, and the like, and combinations thereof. The amount of polyhedric alcohol sweetener used in the suspension will vary depending on the degree of sweetening desired for the particular suspension. Generally, the amount of polyhydric alcohol sweetener will be in the range of from 0 to 90 grams per 100 mL of the suspension.

Suitable flavoring agents include natural and/or artificial flavors such as mints (i.e., peppermint, etc.,), menthol, cinnamon, vanilla, artificial vanilla, chocolate, artificial chocolate, both natural and/or artificial fruit flavors (e.g., cherry, grape, orange, strawberry, etc.,) and combinations of two or more thereof. Flavoring agents are often complex mixtures of chemical compounds dissolved or dispersed in an inert medium, such as, propylene glycol. These solutions or dispersions are generally provided as a minor component of the suspension in amounts effective to provide a palatable flavor to the suspension. However, flavoring agents are generally present in the suspension in amounts in the range of from 0 to 5 grams per 100 mL of the suspension.

Optimum masking of the taste of the solid pharmaceutical active in the suspension can be achieved by limiting the amount of water in the suspension available to solubilize the active agent(s). The minimum amount of water also must provide the suspension with a sufficient aqueous base to impart the desired degree of hydration of the suspending agents. Taste-maskin of bitter pharmaceutical(s) necessitate that the total amount of water contained in the suspension be in the range of from 25 to 60, preferably 30 to 55, grams per 100 mL of suspension.

The pH of the suspension should be optimized to minimize the solubility and maximize the chemical stability of any unpleasant tasting and hydrolysis susceptible active agent, i.e., APAP. as. The pH of the suspension should in the range from 5.1 to 5.9. The suspension can be buffered using pH adjusting agents to maintain the pH of the suspension in the desired pH range. Suitable pH-adjusting agents may be present in the suspension in amounts sufficient to provide the desired degree of pH buffering. The pH-adjusting agents will typically be present in the range of from 0 to 1 gram per 100 mL of the dye-free pharmaceutical suspension. The pH adjusting agent for an embodiment having as an active agent, and including a suspending system having alkaline polymers, such as, sodium carboxymethylcellulose, may be selected from weak organic acids, such as, citric acid, malic acid, glutamic acid, and the like having acceptable taste characteristics for use in tastemasked oral suspensions. Citric acid can be added to the suspension to stabilize the pH of the suspension at between 5.1 to 5.9. Citric acid is advantageously added since this pH range will enhance the stability of the dye-free pharmaceutical suspension. The pH for the suspension when APAP is the pharmaceutical active used is between 5.1 and 5.9 since the APAP will undergo the least degradation in suspension. Antimicrobial preservatives are selected for their activity within this pH range.

Preservatives useful in dye-free pharmaceutical suspensions include, but are not limited to, sodium benzoate, potassium sorbate, salts of edetate (also known as salts of ethylenediaminetetraacetic acid, or EDTA, such as, disodium edetate) and parabens (such as, methyl, ethyl, propyl and butyl p-hydroxybenzoic acids esters). The preservatives listed above are exemplary, but each preservative must be evaluated on an empirical basis, in each formulation, to assure the compatibility and efficacy of the preservative. Methods for evaluating the efficacy of preservatives in pharmaceutical formulations are known to those skilled in the art. The para-aminobenzoic acid dirivitives, e.g., butylparaben, methylparaben, and propylparaben, are particularly useful preservative ingredients to add to a dye-free pharmaceutical suspension containing APAP due to their superior activity in the particularly preferred pH range of from about 5 to about 6. In a particularly useful embodiment, the dye-free pharmaceutical suspension of the present invention is substantially free of benzoic acid and it's derivatives, as these have suboptimal activity in the preferred pH range.

Preservatives are generally present in amounts of up to 1 gram per 100 mL of the suspension. Preferably the preservatives will be present in amounts in the range of from 0.01 to 0.5 gram per 100 mL of the suspension. For dye-free pharmaceutical suspensions containing APAP it is useful that the preservative propylparaben be present in the range of from 0.01 to 0.1 gram per 100 mL of the dye-free pharmaceutical suspension and butylparaben be present in the range of from 0.01 to 0.1 gram per 100 mL of the dye-free pharmaceutical suspension. It is most useful that propylparaben be present at a concentration of 0.045 gram per 100 mL of the dye-free pharmaceutical suspension and butylparaben be present at a concentration of 0. 045 gram per 100 mL of the dye-free pharmaceutical suspension.

The dye-free pharmaceutical suspension of the present invention is substantially free of coloring agents, such as, dyes, or lakes. However, the dye-free pharmaceutical suspension of the present invention may optionally incorporate certain pigments, e.g., titanium dioxide and the like, as opacifiers.

The suspensions also may contain one or more of the following additives defoaming agents, surfactants; electrolytes (monovalent cations are currently preferred); and sequestering agents.

In certain optional embodiments, the dye-free pharmaceutical suspension of the invention may employ a surfactant for use as a wetting agent to aid in the dispersion of certain hydrophobic active agents. In certain other embodiments, the suspension of the invention may be substantially free of surfactant. In embodiments employing a surfactant, one useful surfactant is a sorbitan oleate ester, particularly, polyoxyethylene sorbitan monooleate also known as polysorbate 80.

The suspensions of the present invention can employ suspending systems as known in the art that include, but are not limited to, at least one thickening component. The thickening component typically includes one or more thickening agents that may be selected from hydrophilic, i.e., water souble, polymers such as hydrocolloids, swelling or gelling polymers, and the like. In one embodiment, the thickening component combines the attributes of a structuring agent and a swelling agent. In another preferred embodiment, the thickening component combines the attributes of at least two structuring agents, e.g., a primary structuring agent and a secondary structuring agent.

A structuring agent, when introduced into an appropriate aqueous environment, forms an ordered structure, believed to be stabilized by hydrogen bonding and molecular entanglement. Hydrocolloids are a particularly good type of structuring agent. Hydrocolloids are dispersions of particles around which water molecules and solvated ions form a shell-like structure, fluid absorption occurs principally by swelling and enlargement of the structure.

Examples of suitable hydrocolloids include, but are not limited to, alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, karaya, whelan, rhamsan, zooglan, methylan, chitin, cyclodextrin, chitosan, cellulosic polymers such as microcrystalline cellulose, carboxymethylcellulose, and derivatives and combinations thereof. In certain embodiments of the present invention, useful structuring agents may be selected from the hydrocolloids xanthan gum, microcrystalline cellulose, carboxymethylcellulose, and derivatives, co-precipitates, and combinations thereof. In one particularly useful embodiment, the thickening component inlcudes xanthan gum as a primary structuring agent and a co-processed combination of microcrystalline cellulose and carboxymethylcellulose (such as that commercially available from FMC as Avicel-RC 591) as a secondary structuring agent.

Xanthan gum is a high molecular weight natural carbohydrate, specifically, a polysaccharide. The xanthan gum suitable for use in the present invention is a high molecular weight polysaccharide produced by Xanthomonas campestris. Techniques and strains for producing this polysaccharide are described in U.S. Pat. Nos. 4,752,580 and 3,485,719. The xanthan gum used in the present invention should have a viscosity in a one percent salt solution of from 1000 to 1700 cP (mPa-sec). The one percent solution's viscosity should be measured at 25 °C with an LV model Brookfield Synchro-Lectric viscometer at 60 rpm, no. 3 spindle. Xanthan gum is available from several commercial suppliers such a RT Vanderbilt Company and CP Kelco. Examples of suitable xanthan gums are Keltrol, Keltrol F, Keltrol T, Keltrol TF, Xantural 180 and Vanzan NF-ST.

In a useful embodiment, the secondary structuring agent used in the present invention is a dried coprecipitated microcrystal of cellulose and sodium carboxymethylcellulose. Sodium carboxymethyl-cellulose is commonly used as a coprecipitate in microcrystalline cellulose. It is particularly useful if the sodium carboxymethylcellulose is included in the range of from 8 weight percent to 19 weight percent of the total weight of the coprecipitated microcrystal of cellulose and sodium carboxymethylcellulose, Useful are microcrystalline cellulose products having in the range from 8 to 14 weight percent sodium carboxymethylcellulose. These mixtures as described above are commercially available from a variety of sources, including FMC under the trademark Avicel^{®} CL-611, Avicel^{®} RC-581 and Avicel^{®} RC-591.

The thickening component may optionally comprise a swelling agent, when exposed to an appropriate aqueous environment, expands and may interact with the structuring agent. Pregelatinized starch is a particularly good swelling agent. Pregelatinized starch, also known as "instantized" starch, is precooked so that it swells and begins to thicken instantly when added to cold water. ne particularly suitable pregelatinized starch is prepared from modified, stabilized and waxy, maize food starch, and commercially available from National Starch Company as Instant Starch, Ultrasperse M.

In certain embodiments, an optional auxiliary suspending agent used in the present invention. The auxiliary suspending agent may be selected from the group consisting of hydroxyethylcellulose and a pharmaceutically acceptable salt of carboxymethylcellulose. Suitable pharmaceutically acceptable salts of carboxymethylcellulose include sodium and calcium salts of a polycarboxymethyl ether of cellulose, commercially available as sodium carboxymethylcellulose, USP and calcium carboxymethylcellulose, NF. Sodium carboxymethylcellulose, USP contains between 6.5 to 7.5% by weight sodium on a dry basis and is commercially available from Aqualon Co. under the product designation Aqualon. The hydroxyethylcellulose is a partially substituted poly(hydroxyethyl) ether of cellulose. Hydroxycellulose, NF is commercially available from Aqualon Co. under the product designation Natrosol.

A useful process for preparing the aqueous pharmaceutical suspension composition includes the following sequential steps:
(a) adding from 35 to 40 weight percent of water to achieve suitable volume for mixing;
(b) dispersing from 0.5 to 1.0% microcrystalline cellulose and carboxymethylcellulose (co-processed) and from 0.1 to 0.2% xanthan gum and mixing until hydrated;
(c) adding from 10 to 50% sorbitol, by weight by volume of the total suspension followed by 5 to 20% glycerin;
(d) adding from 20 to 50% sucrose, by weight by volume of the total suspension to the dispersion of step (c) and mixing until the ingredients are uniformly dispersed in the mixture;
(e) adding sufficient citric acid anhydrous powder to lower the pH of the solution to between 5.1 to 5.9 to the mixture of step (d) until the ingredients are uniformly dispersed throughout the mixture;or in other embodiments, sodium citrate anhydrous powder is added to adjust the pH;
(f) adding a mixture of 0.03 to 0.06% propylparaben and from 0.03 to 0.06% butylparaben solubilized in 0.1 to 1.0% propylene glycol;
(g) adding the first active agent, e.g., from 3 to 12% APAP, followed by the flavoring system from 0.05 to 0.15% and suitable high intensity sweetener, e.g., from 0.1 to 0.2% sucralose; and
(h) adding and mixing sufficient water to the mixture of step (g) to produce an dye-free pharmaceutical suspension of 100% desired volume.

In useful embodiments of the process an effective amount of preservative, such as, for example, propylparaben and butylparaben, is added to the mixture in step (f) and the suspension in step (h) is subjected to a deaerating step so that the volume of the suspension is adjusted to 100% by addition of water after such deaerating. The flavoring ingredients added to the mixture in step (g) may be of the type and amount desired for the particular suspension to meet the preferences dictated by the intended consumer of such suspension e.g., pediatric or adult. A more detailed example of a useful process of the present invention is provided in the following examples.

Suspension viscosity is measured using a Brookfield LV Viscometer equipped with Spindle #31. Sample from an unopened bottle was dispensed into the sample chamber and equilibrated in a water bath to 25°C. After equilibration, sample was stirred at 1.5 rpm and viscosity read after 2 minutes.

Useful viscosity of the suspension of the present invention is from 1500 to 7000 centipoise, e.g. not less than 1800 centipoise, or not less than 2300 centipoise when measured according to the above method.

### EXAMPLES

The invention will now be illustrated by examples. The examples are not intended to be limiting of the scope of the present invention but read in conjunction with the detailed and general description above, provide further understanding of the present invention and an outline of a preferred process for preparing the compositions of the invention.

### Example 1

### Dye-Free APAP Suspension

| Ingredient | Amount (%w/v) | % Solids |
|---|---|---|
| Purified Water, USP | 45 | 0 |
| Sorbitol Solution, USP 70% | 20 | 14 |
| Microcrystalline Cellulose and Carboxymethylcellulose (co-processed) NF | 0.70 | 0.70 |
| Xanthan Gum NF | 0.14 | 0.14 |
| Glycerin USP | 10 | 10 |
| Sucrose NF | 45 | 45 |
| Citric Acid USP Anhydrous | 0.006 | 0.006 |
| Propylene Glycol USP | 0.25 | 0.25 |
| Butylparaben NF | 0.045 | 0.0125 |
| Propylparaben NF | 0.045 | 0.045 |
| Acetaminophen USP, Fine Powder | 3.2 | 3.2 |
| Sucralose liquid concentrate | 0.30 | 0.075 |
| Flavoring agents | 0.07 | 0.34 |
| | | |
| TOTAL | 100 mL | 74% solids 51% water |

### PROCESSING DIRECTIONS

1. To a tared vessel equipped with high shear vacuum mixer, add from about 35 to about 40 weight percent of purified water to achieve suitable volume for mixing.
2. Disperse from about 0.5 to about 1.0% microcrystalline cellulose and carboxymethylcellulose (co-processed) and from about 0.1 to about 0.2% xanthan gum and mix until hydrated.
3. Add sorbitol solution followed by glycerin and mix.
4. Add sucrose and mix until dissolved.
5. Add citric acid anhydrous powder followed by propylparaben and butylparaben predissolved in propylene and mix until dissolved.
6. Disperse APAP followed by sucralose and flavor system and mix.
7. Bring suspension to final volume with purified water, and mix under vacuum to deaerate.

The above produces a batch size of approximately 7570 liters or 2000 gallons of dye-free acetaminophen suspension (100 mg/5 ml) at a pH of 5.5.

### Example 2

### Dye-free APAP Suspension Drops

| Ingredient | Amount (%w/v) | % Solids |
|---|---|---|
| Purified Water, USP | 36 | 0 |
| Sorbitol Solution, USP 70% | 62 | 43.4 |
| Microcrystalline Cellulose and Carboxymethylcellulose (co-processed) NF | 0.70 | 0.70 |
| Xanthan Gum NF | 0.14 | 0.14 |
| Glycerin USP | 10 | 10 |
| | | |
| Citric Acid USP Anhydrous | 0.002 | 0.002 |
| Propylene Glycol USP | 0.25 | 0.25 |
| Butylparaben NF | 0.045 | 0.0125 |
| Propylparaben NF | 0.045 | 0.045 |
| Acetaminophen USP, Fine Powder | 10 | 10 |
| Sucralose liquid concentrate | 0.50 | 0.125 |
| Flavoring Agents | 0.082 | 0.082 |
| | | |
| TOTAL | 100 mL | 65% solids 55% water |

### PROCESSING DIRECTIONS

1. To a tared vessel equipped with high shear vacuum mixer, add about 35 weight percent of purified water and about 24 weight percent sorbitol solution to achieve suitable volume for mixing.
2. Disperse from about 0.5 to about 1.0% microcrystalline cellulose and carboxymethylcellulose (co-processed) and from about 0.1 to about 0.2% xanthan gum and mix until hydrated.
3. Add remaining sorbitol solution followed by glycerin and mix.
4. Add citric acid anhydrous powder followed by propylparaben and butylparaben predissolved in propylene and mix until dissolved.
5. Add APAP followed by sucralose and flavor system and mix dispersed.
6. Bring suspension to final volume with purified water, and mix under vacuum to deaerate.

The above produces a batch size of 3785 Liters dye-free acetaminophen suspension drops (80mg/0.8mL) at a pH of 5.5.

### Example 3

### Dye-Free APAP, Dextromethorphan HBr and Pseudoephedrine HCl Suspension Drop Dosage Form

| Ingredient | Amount (%w/v) |
|---|---|
| Purified Water, USP | 35 |
| Sorbitol Solution, USP 70% | 62 |
| Microcrystalline Cellulose and Carboxymethylcellulose (co-processed) NF | 0.90 |
| Dextromethorphan HBr USP | 0.3125 |
| Xanthan Gum NF | 0.18 |
| Glycerin USP | 5 |
| | |
| Sodium Citrate USP Anhydrous | 0.018 |
| Propylene Glycol USP | 0.25 |
| Butylparaben NF | 0.045 |
| Propylparaben NF | 0.045 |
| Pseudoephedrine HCl USP | 0.9375 |
| Acetaminophen USP, Fine Powder | 10 |
| Sucralose liquid concentrate | 0.60 |
| Flavoring Agents | 0.092 |
| TOTAL | 100 mL |

The process of Example 2 is carried out except that the dextromethorphan hydrobromide is added and mixed to dissolve in between the hydration of the microcrystalline cellulose and carboxymethylcellulose (co-processed) and the xanthan gum. The pseudoephedrine hydrochloride is added and mixed to dissolve just prior to the addition of the acetaminophen. Sodium citrate anhydrous powder is added in place of citric acid anhydrous powder for pH adjustment.

### Example 4

### Dye-Free APAP, Dextromethorphan HBr, Pseudoephedrine HCl and Chlorpheniramine

### Maleate Suspension Dosage Form

| Ingredient | Amount (%w/v) |
|---|---|
| Purified Water, USP | 44 |
| Sorbitol Solution, USP 70% | 20 |
| Microcrystalline Cellulose and Carboxymethylcellulose (co-processed) NF | 0.70 |
| Dextromethorphan HBr USP | 0.10 |
| Xanthan Gum NF | 0.14 |
| Glycerin USP | 10 |
| Sucrose NF | 45 |
| Propylene Glycol USP | 0.25 |
| Butylparaben NF | 0.045 |
| Propylparaben NF | 0.045 |
| Cholorpheniramine Maleate USP | 0.02 |
| Pseudoephedrine HCl USP | 0.30 |
| Acetaminophen USP, Fine Powder | 3.2 |
| Sucralose liquid concentrate | 0.40 |
| Flavoring Agents | 0.08 |
| TOTAL | 100 mL |

The process of Example 1 is carried out except that the dextromethorphan hydrobromide is added and mixed to dissolve in between the hydration of the microcrystalline cellulose and carboxymethylcellulose (co-processed) and the xanthan gum. The chlorpheniramine maleate and pseudoephedrine hydrochloride are added and mixed to dissolve just prior to the addition of the acetaminophen.

### Example 5

### Comparison of HFCS-containing suspensions and suspensions substantially-free of reducing sugars

### Dye-Free Acetaminophen, Dextromethorphan HBr and Pseudoephedrine HCl_Suspension Drops

| Ingredient | Amount (%w/v) Substantially Reducing Sugar-Free | Amount (%w/v) 5% Reducing Sugar | Amount (%w/v) 10% Reducing Sugar | Amount (%w/v) 20% Reducing Sugar |
|---|---|---|---|---|
| Purified Water, USP | 38 | 38 | 38 | 38 |
| Sorbitol Solution, USP 70% | 62 | 57 | 52 | 42 |
| High Fructose Corn Syrup 55% | 0 | 5 | 10 | 20 |
| Microcrystalline Cellulose and Carboxymethylcellulose (co-processed) NF | 0.90 | 0.90 | 0.90 | 0.90 |
| Dextromethorphan HBr USP | 0.3125 | 0.3125 | 0.3125 | 0.3125 |
| Xanthan Gum NF | 0.18 | 0.18 | 0.18 | 0.18 |
| Glycerin USP | 5 | 5 | 5 | 5 |
| Sodium Citrate USP Anhydrous | 0.018 | 0.018 | 0.018 | 0.018 |
| Propylene Glycol USP | 0.25 | 0.25 | 0.25 | 0.25 |
| Butylparaben NF | 0.045 | 0.045 | 0.045 | 0.045 |
| Propylparaben NF | 0.045 | 0.045 | 0.045 | 0.045 |
| Pseudoephedrine HCl USP | 0.9375 | 0.9375 | 0.9375 | 0.9375 |
| Acetaminophen USP, Fine Powder | 10 | 20 | 10 | 10 |
| Sucralose liquid concentrate | 0.60 | 0.60 | 0.60 | 0.60 |
| Flavoring Agents | 0.092 | 0.092 | 0.092 | 0.092 |
| | | | | |
| TOTAL | 100 mL | 100 mL | 100 mL | 100 mL |

The process of Example 3 is carried out except that in the suspensions that contain the various levels of High Fructose Corn Syrup 55%, a portion of Sorbitol Solution USP 70% is removed to allow the addition. The 4 samples were packaged and placed under stress conditions of 40°C and 50°C for several weeks. After observing the samples pulled from the stress conditions at various time periods, e.g., 1-week, 2-weeks, etc., it was noted that a level of discoloration developed in the samples containing High Fructose Corn Syrup 55% were increased.

### Discoloration of temperature stressed samples in Dye-Free Acetaminophen, Dextromethorphan HBr and Pseudoephedrine HCl Suspension Drops

### Comparison of HFCS-containing suspensions and suspensions substantially-free of reducing sugars

| Color Observation * | Substantially Reducing Sugar-Free | 5% w/v Reducing Sugar | 10% w/v Reducing Sugar | 20% w/v Reducing Sugar |
|---|---|---|---|---|
| Stressed 1-month @ 50°C | 3 | 4 | 4 | 5 |
| Stressed 2-months @ 40°C | 2 | 3 | 3 | 4 |

| | | | | |
|---|---|---|---|---|
| * Color Observations: NC: No Change 1: Very slight change, noticeable to a trained analyst 2: Definite change, noticeable to a trained analyst 3: Change, barely noticeable to a consumer 4: Definite change, noticeable to a consumer 5: Extreme change | | | | |

The suspensions containing reducing sugar at a level as low as 5%w/v show noticeably more discoloration that the preferred suspension with no reducing sugar.

The scope of the present invention is not limited by the description, examples and suggested uses herein and modifications can be made without departing from the spirit of the invention. For example, additional medicaments may be added to the aqueous suspension to provide a combination medication. Further, the pharmaceutical suspension of the invention may be utilized for non-medicament ingredients including nutrients such as vitamins and minerals. Application of the compositions and method of the present invention for medical and pharmaceutical uses can be accomplished by any clinical, medical and pharmaceutical methods and techniques as are presently or prospectively known to those skilled in the art. Thus it is intended that the present invention cover the modifications and variations of this invention provided that they come within the scope of the appended claims and their equivalents.

## Claims

1. A dye-free acetaminophen (APAP) suspension, comprising:
(a) a therapeutically effective amount of APAP having an average particle size of between about 10 and about 100 microns,
(b) an effective amount of a non-reducing sweetener comprising a combination of sucrose and sorbitol;
(c) from 25 to 60 grams per 100 mL of the suspension of water;
(d) an effective amount of a suspending system;
wherein the dye-free pharmaceutical suspension has a pH of from 5.1 to 5.9 and is substantially free of a reducing sugar.

2. The dye-free acetaminophen suspension of claim 1, additionally comprising titanium dioxide.

3. The dye-free acetaminophen suspension of any preceding claim, wherein the suspension further comprises a therapeutically effective amount of a second active agent selected from the group consisting of a second substantially water insoluble active agent, a water soluble active agent, or mixtures thereof.

4. The dye-free acetaminophen suspension of claim 3, wherein the second active agent is selected from the group consisting of an antitussive, an expectorant, an antihistamine, a sympathomimetic, and mixtures thereof.

5. The dye-free acetaminophen suspension of any preceding claim having a viscosity from 1500 to 7000 centipoise.

## Patentansprüche

1. Farbstofffreie Acetaminophen(APAP)-Suspension, die Folgendes umfasst:
(a) eine therapeutisch wirksame Menge an APAP mit einer durchschnittlichen Teilchengröße von zwischen ungefähr 10 und ungefähr 100 Mikrometern,
(b) eine wirksame Menge eines nichtreduzierenden Süßstoffs, der eine Kombination aus Saccharose und Sorbit umfasst;
(c) 25 bis 60 Gramm Wasser pro 100 ml Suspension;
(d) eine wirksame Menge eines Suspendiersystems;
wobei die farbstofffreie pharmazeutische Suspension einen pH-Wert von 5,1 bis 5,9 aufweist und im Wesentlichen frei von einem reduzierenden Zucker ist.

2. Farbstofffreie Acetaminophensuspension nach Anspruch 1, die zusätzlich Titandioxid umfasst.

3. Farbstofffreie Acetaminophensuspension nach einem vorhergehenden Anspruch, wobei die Suspension weiterhin eine therapeutisch wirksame Menge eines zweiten Wirkstoffs, ausgewählt aus der Gruppe bestehend aus einem zweiten im Wesentlichen wasserunlöslichen Wirkstoff, einem wasserlöslichen Wirkstoff oder Mischungen davon, umfasst.

4. Farbstofffreie Acetaminophensuspension nach Anspruch 3, wobei der zweite Wirkstoff aus der Gruppe bestehend aus einem Antitussivum, einem Expectorans, einem Antihistamin, einem Sympathomimetikum und Mischungen davon ausgewählt ist.

5. Farbstofffreie Acetaminophensuspension nach einem vorhergehenden Anspruch mit einer Viskosität von 1500 bis 7000 Centipoise.

## Revendications

1. Suspension d'acétaminophène (APAP) sans colorant, comprenant :
(a) une quantité thérapeutiquement efficace d'APAP ayant une taille moyenne de particules comprise entre environ 10 et environ 100 microns ;
(b) une quantité efficace d'un édulcorant non réducteur comprenant une combinaison de saccharose et de sorbitol ;
(c) de 25 à 60 grammes, pour 100 ml de la suspension, d'eau ;
(d) une quantité efficace d'un système de mise en suspension ;
où la suspension pharmaceutique sans colorant possède un pH allant de 5,1 à 5,9 et est essentiellement dépourvue d'un sucre réducteur.

2. Suspension d'acétaminophène sans colorant selon la revendication 1, comprenant en outre du dioxyde de titane.

3. Suspension d'acétaminophène sans colorant selon l'une quelconque des revendications précédentes, où la suspension comprend en outre une quantité thérapeutiquement efficace d'un deuxième agent actif choisi dans le groupe constitué par un deuxième agent actif sensiblement insoluble dans l'eau, un agent actif soluble dans l'eau, ou des mélanges de ceux-ci.

4. Suspension d'acétaminophène sans colorant selon la revendication 3, dans laquelle le deuxième agent actif est choisi dans le groupe constitué par un agent antitussif, un agent expectorant, un agent antihistaminique, un agent sympathomimétique, et des mélanges de ceux-ci.

5. Suspension d'acétaminophène sans colorant selon l'une quelconque des revendications précédentes, ayant une viscosité allant de 1500 à 7000 centipoises.
